# EUROPEAN PATENT APPLICATION

(11) **EP 4 364 757 A2**
(43) Date of publication of application: **08.05.2024**
(21) Application number: 23192928.2
(22) Date of filing: 23.08.2023
(51) Int. Cl.: A61L 2/07, A61L 2/26, F04D 29/00, F26B 5/04

(54) **STEAM STERILIZER AND STERILIZATION DRYING METHOD THEREOF**

(30) Priority: 04.11.2022 CN 202211374430
(71) Applicant: Ningbo Haishu YESON Medical Device Co., Ltd., 315181 Ningbo Zhejiang (CN)
(72) Inventor: LU, Lihui, Ningbo, 315181 (CN); SHEN, Yuanye, Ningbo, 315181 (CN); ZHENG, Kejie, Ningbo, 315181 (CN)
(74) Representative: Spengler, Robert

(57) **Abstract**

A steam sterilizer is provided which comprises a sterilizer body, and a drying system disposed inside the sterilizer body. The drying system comprises an autoclave, a heating apparatus, a steam apparatus, a vacuuming apparatus and a return air apparatus, where the autoclave is connected with the heating apparatus, the steam apparatus, the vacuuming apparatus and the return air apparatus. The heating apparatus is used to heat the autoclave; the steam apparatus is used to fill steam into the autoclave; the vacuuming apparatus is used to vacuum the autoclave; and the return air apparatus is used to return air into the vacuumed autoclave. The sterilizer body is internally provided with an air-blowing apparatus used to stir an air current in the autoclave so that the air current in the autoclave can be stirred in a drying process; in a drying process, the heating apparatus heats the autoclave and the heat on the autoclave can be more easily transferred to a load under the action of flowing air, so as to speed up water evaporation. In this way, the drying efficiency and effect can be improved without increasing the vacuuming degree or the heating temperature or extending the drying time.

## Description

### TECHNICAL FIELD

The present disclosure relates to the field of sterilizer technologies and in particular to a steam sterilizer and a sterilization drying method thereof.

### BACKGROUND

Some medical instruments, after being used, should be placed into a steam sterilizer for sterilization and disinfection, so as to eliminate all bacteria and viruses on the medical instruments and keep these medical instruments clean and hygienic, avoiding cross infection of patients. The sterilization process of the steam sterilizer usually comprises the following steps: firstly, by using a vacuuming apparatus of the steam sterilizer, extracting the air in the autoclave and then, injecting high-temperature and high-pressure steam into the autoclave and holding the high-temperature and high-pressure steam for a period of time to achieve the sterilization effect; finally, by using the vacuuming apparatus, extracting the air and water stream in the autoclave. In this process, a heating apparatus outside the autoclave may heat the autoclave to speed up water evaporation in the autoclave until the load (i.e. medical instrument in the autoclave) is fully dried.

In actual use, the loads have extremely high dry requirements. If wet pack is present in the loads (i.e. not dry), re-contamination may occur after sterilization. During the vacuuming process of the steam sterilizer, the water boiling point will be lowered and the heating apparatus will more easily vaporize the water to achieve the drying purpose. But, in the vacuuming process, the air or water steam in the autoclave may gradually become thin whereas a heating ring on the heating apparatus heats the autoclave, which leads to difficulty in transferring heat to the loads as well as occurrence of non-drying phenomenon. In order to avoid the occurrences, it is usually required to increase the vacuuming degree or increase the temperature of the heating ring or increase the drying time to improve the drying degree. Howevet, these methods may easily shorten the service life of the vacuuming apparatus and heating apparatus and may even bring damage to the loads.

### SUMMARY

In order to overcome the defects in the prior arts, the present disclosure provides a quickly-vacuumed steam sterilizer and a sterilization drying method thereof.

The technical solution of the present disclosure is to provide a steam sterilizer with the following structure: comprising a sterilizer body, and a drying system disposed inside the sterilizer body. The drying system comprises an autoclave, a heating apparatus, a steam apparatus, a vacuuming apparatus and a return air apparatus, where the autoclave is connected with the heating apparatus, the steam apparatus, the vacuuming apparatus and the return air apparatus. The heating apparatus is used to heat the autoclave; the steam apparatus is used to fill steam into the autoclave; the vacuuming apparatus is used to vacuum the autoclave; and the return air apparatus is used to return air into the vacuumed autoclave. The sterilizer body is internally provided with an air-blowing apparatus used to stir an air current in the autoclave.

Compared with the prior arts, the steam sterilizer with the above structure in the present disclosure has the following advantages:
With the air-blowing apparatus disposed in the autoclave, the air current in the autoclave can be stirred in a drying process; in a drying process, the heating apparatus heats the autoclave and the heat on the autoclave can be more easily transferred to a load under the action of flowing air, so as to speed up water evaporation. In this way, the drying efficiency and effect can be improved without increasing the vacuuming degree or the heating temperature or extending the drying time.

Preferably, the air-blowing apparatus comprises a drive unit and fan blades. The drive unit is disposed outside the autoclave and the fan blades are rotatably connected inside the autoclave. The drive unit is connected with the fan blades in transmission manner to drive the rotation of the fan blades.

Preferably, a first magnetic coupling is rotatably connected outside the autoclave and a second magnetic coupling is rotatably connected at a position corresponding to the first magnetic coupling inside the autoclave. The drive unit is connected to the first magnetic coupling and the second magnetic coupling is connected to the fan blades. The drive unit is used to drive the first magnetic coupling to rotate, and the first magnetic coupling is magnetically attracted with the second magnetic coupling to drive the second magnetic coupling and the fan blades to rotate. The non-contact transmission between the drive unit and the fan blades, which is achieved by magnetic attraction of the first magnetic coupling and the second magnetic coupling, will not damage the sealing performance of the autoclave, thereby avoiding contamination.

Preferably, each of the first magnetic coupling and the second magnetic coupling comprises a base, a magnet and a rotary shaft. The base is fixedly mounted on the autoclave. A bearing is disposed on the base. The rotary shaft is rotatably connected to the bearing. The magnet is connected to the rotary shaft to rotate along with the rotary shaft.

Preferably, there are six magnets which are annularly distributed on an outer wall of the rotary shaft. In this case, the transmission between the first magnetic coupling and the second magnetic coupling is made more stable.

Preferably, the autoclave is an autoclave made of a non-magnetic material. The autoclave made of the non-magnetic material can avoid influence on the magnets and increase the transmission effect between the first magnetic coupling and the second magnetic coupling.

Preferably, the magnets are magnets resistant to high temperature, and the bearing on the second magnetic coupling is a ceramic bearing. Since the heating apparatus can heat the autoclave, the high-temperature resistant magnets can better adapt so as to avoid affecting the magnetism. The ceramic bearing has smooth surface and good wear resistance and can be used in the oil-free autoclave with rich water steam and high temperature.

Preferably, the fan blades are externally sleeved with a protective hood with hollow design. When a load is placed by a user, the protective hood prevents the load from being in contact with the fan blades, ensuring rotation of the fan blades.

The present disclosure further provides a sterilization drying method for the steam sterilizer, which includes the following steps:
at step 1, preheating stage: the heating apparatus heats and controls the outer wall of the autoclave to a particular temperature, and meanwhile the air-blowing apparatus stirs the air current in the autoclave until the temperature in the autoclave reaches a preset temperature;
at step 2, sterilization stage: the vacuuming apparatus vacuums the autoclave, and then the steam apparatus fills high-temperature water steam into the autoclave and holds for a period of time to achieve sterilization effect;
at step 3, water and air drainage stage: the vacuuming apparatus vacuums the autoclave to drain air and water steam in the autoclave;
at step 4, drying stage: after the vacuuming apparatus vacuums the autoclave to a set vacuum degree, the vacuuming apparatus is turned off, and the return air apparatus is turned on to allow a large amount of bacteria-filtered air to enter the autoclave; the air in the autoclave is stirred to circulate by the air-blowing apparatus, such that the heating apparatus quickly heats the interior of the autoclave and a load in the autoclave until the temperature in the autoclave reaches a set temperature, and then the vacuuming apparatus is turned on again to perform vacuuming;
at step 5, the operation of step 4 is repeated until the load is fully dried.

In this method, the autoclave is preheated by using the heating apparatus to reduce condensation of the water steam filled in sterilization stage; during a drying process, the air-blowing apparatus stirs the air current in the autoclave such that the heat on the autoclave can be more easily transferred to the load under the action of the flowing air, so as to speed up water evaporation. Therefore, the drying efficiency and effect can be improved without increasing the vacuuming degree or heating temperature or extending the drying time.

### BRIEF DESCRIPTIONS OF THE DRAWINGS

FIG. 1 is a structural schematic diagram of the present disclosure.
FIG. 2 is a schematic diagram illustrating a partial structure of a drying system according to the present disclosure.
FIG. 3 is a partial sectional view of a drying system according to the present disclosure.
FIG. 4 is a semi-sectional view of an air-blowing apparatus according to the present disclosure.

Numerals of the present disclosure is described below: 1. sterilizer body, 2. autoclave, 3. air-blowing apparatus, 4. drive unit, 5. fan blade, 6. first magnetic coupling, 7. second magnetic coupling, 8. base, 9. magnet, 10. rotary shaft, 11. protective hood, 12. bearing.

### DETAILED DESCRIPTIONS OF EMBODIMENTS

The present disclosure will be further detailed below in combination with accompanying drawings and specific embodiments.

In the descriptions of the present disclosure, it should be understood that the orientation or positional relationship indicated by the terms such as "upper", "lower", "front", "rear", "left", "right", "vertical", "horizontal", "top", "bottom", "inside", and "outside" is based on the orientation or positional relationship shown in accompanying drawings and used only for ease of descriptions and simplification of descriptions and does not indicate or imply that the indicated devices or elements must have a particular orientation, or be constructed or operated in a particular orientation. Therefore, such terms shall not be understood as limiting of the present disclosure. Furthermore, the terms such as "first", and "second" are used only to distinguish the names of the components without priority and thus shall not be understood as limiting of the present disclosure.

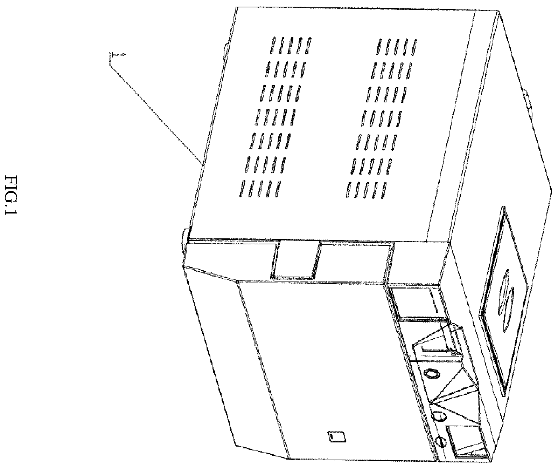

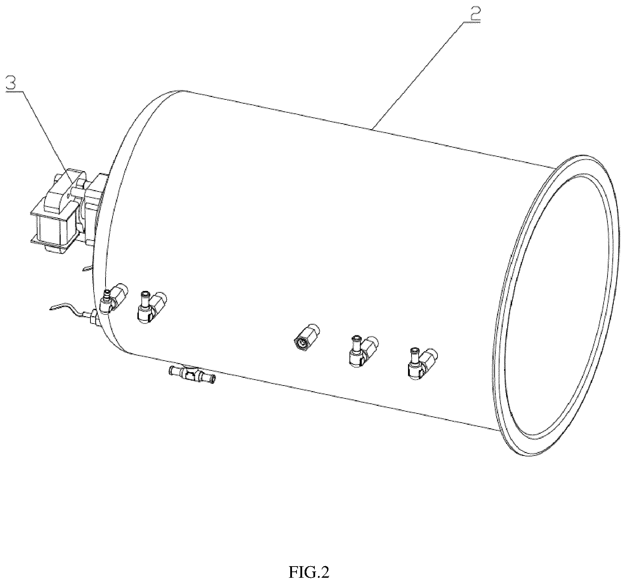

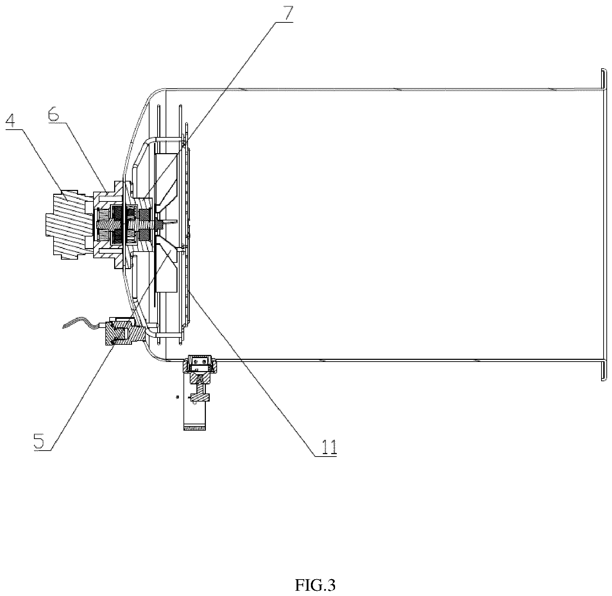

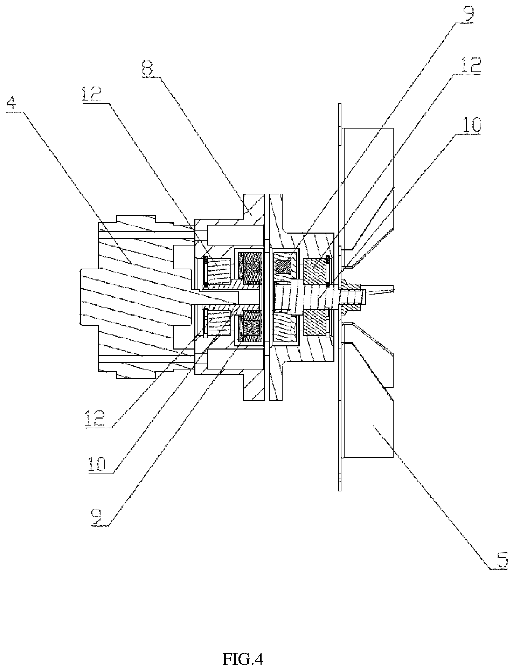

With reference to FIGS. 1 to 4, the present disclosure provides a steam sterilizer, which comprises a sterilizer body 1, and a drying system disposed inside the sterilizer body 1. The drying system comprises an autoclave 2, a heating apparatus, a steam apparatus, a vacuuming apparatus and a return air apparatus, where the autoclave 2 is connected with the heating apparatus, the steam apparatus, the vacuuming apparatus and the return air apparatus. The heating apparatus is used to heat the autoclave 2; the steam apparatus is used to fill steam into the autoclave 2; the vacuuming apparatus is used to vacuum the autoclave 2; and the return air apparatus is used to return air into the vacuumed autoclave 2.

The sterilizer body 1 is internally provided with an air-blowing apparatus 3 used to stir an air current in the autoclave 3. In a drying process, the heating apparatus heats the autoclave 2 and meanwhile the air-blowing apparatus stirs the air current in the autoclave 2. Thus, the heat on the autoclave 2 can be more easily transferred to a load under the action of flowing air, so as to speed up water evaporation. In this way, the drying efficiency and effect can be improved without increasing the vacuuming degree or the heating temperature or extending the drying time.

The air-blowing apparatus 3 comprises a drive unit 4 (e.g. drive motor) and fan blades 5. The drive unit 4 is disposed outside the autoclave 2 and the fan blades are rotatably connected inside the autoclave 2. The drive unit 4 is connected with the fan blades 5 in transmission manner to drive the rotation of the fan blades 5.

A first magnetic coupling 6 is rotatably connected outside the autoclave 2 and a second magnetic coupling 7 is rotatably connected at a position corresponding to the first magnetic coupling 6 inside the autoclave 2. The drive unit 4 is connected to the first magnetic coupling 6 and the second magnetic coupling 7 is connected to the fan blades 5. The drive unit 4 is used to drive the first magnetic coupling 6 to rotate, and the first magnetic coupling 6 is magnetically attracted with the second magnetic coupling 7 to drive the second magnetic coupling 7 and the fan blades 5 to rotate. The transmission between the drive unit 4 and the fan blades 5, which is achieved by magnetic attraction of the first magnetic coupling 6 and the second magnetic coupling 7, prevents the drive unit 4 from contacting with the interior of the autoclave 2, thereby avoiding contamination.

Each of the first magnetic coupling 6 and the second magnetic coupling 7 comprises a base 8, a magnet 9 and a rotary shaft 10. The base 8 is fixedly mounted on the autoclave 2. A bearing 12 is disposed on the base 8. The rotary shaft 10 is rotatably connected to the bearing 12. The magnet 9 is connected to the rotary shaft 10 to rotate along with the rotary shaft 10. There are six magnets 9 which are annularly distributed on an outer wall of the rotary shaft 10. In this case, the transmission between the first magnetic coupling 6 and the second magnetic coupling 7 is made more stable.

The autoclave 2, the base 8 and the rotary shaft 10 are all made of a non-magnetic material, for example, aluminum alloy or stainless steel or the like. The autoclave 2, the base 8 and the rotary shaft 10 which are made of a non-magnetic material can avoid influence on the magnets 9 and increase the transmission effect between the first magnetic coupling 6 and the second magnetic coupling 7.

The magnets 9 are magnets resistant to high temperature, and the bearing 12 on the second magnetic coupling 7 is a ceramic bearing. Since the heating apparatus can heat the autoclave 2, the high-temperature resistant magnets can be better adapted to avoid affecting the magnetism. The ceramic bearing has smooth surface and good wear resistance and can be used in the oil-free autoclave 2 with rich water steam and high temperature. No lubricant is required and thus the load in the autoclave 2 will not be contaminated.

The fan blades are externally sleeved with a protective hood 11 with hollow design. When a load is placed by a user, the protective hood 11 prevents the load from being in contact with the fan blades 5, ensuring rotation of the fan blades 5.

The heating apparatus comprises a heating ring, a temperature detector and a temperature control switch. The heating ring is disposed on the autoclave 2 to heat the autoclave 2. The temperature detector is used to detect a temperature of the autoclave 2 and transmit a detected signal to the temperature control switch which is used to turn on or off the heating ring. The steam apparatus comprises a water tank and a steam generator. The vacuuming apparatus comprises a vacuum pump, an air exhaust pipe, an air exhaust valve disposed on the air exhaust pipe, a condenser and a water-air separator. The condenser is used to condense the steam flowing through the air exhaust pipe into water and the water-air separator is used to separate water and air in the air exhaust pipe. The vacuum pump is in communication with an accommodation chamber through the air exhaust pipe to pump out the condensate and water steam in the accommodation chamber. The return air apparatus comprises an air filter, an air inlet pipe and an air inlet valve disposed on the air inlet pipe. The above descriptions belong to the prior arts as disclosed by the patent publication number CN111888492A titled a sterilizer, in which a heating apparatus, an air exhaust mechanism and an air supply mechanism are disclosed. Therefore, no detailed descriptions are made herein.

The air-blowing apparatus 3 may also be a blower. An air inlet of the blower is provided with an air filter and an air outlet of the blower is in communication with the autoclave 2. The air outlet of the blower is further provided with an electromagnetic valve switch which can close the air outlet in the sterilization stage and open the air outlet in the drying stage.

The present disclosure further provides a sterilization drying method for the steam sterilizer, which includes the following steps:
at step 1, preheating stage: the heating apparatus heats and controls the outer wall of the autoclave to a particular temperature, and meanwhile the air-blowing apparatus stirs the air current in the autoclave until the temperature in the autoclave reaches a preset temperature;
at step 2, sterilization stage: the vacuuming apparatus vacuums the autoclave several times, and then the steam apparatus fills high-temperature water steam into the autoclave and holds for a period of time to achieve sterilization effect;
at step 3, water and air drainage stage: the vacuuming apparatus vacuums the autoclave to drain air and water steam in the autoclave;
at step 4, drying stage: after the vacuuming apparatus vacuums the autoclave to a set vacuum degree, the vacuuming apparatus is turned off, and the return air apparatus is turned on to allow a large amount of bacteria-filtered air to enter the autoclave; the air in the autoclave is stirred to circulate by the air-blowing apparatus, such that the heating apparatus quickly heats the interior of the autoclave and a load in the autoclave until the temperature in the autoclave reaches a set temperature, and then the vacuuming apparatus is turned on again to perform vacuuming;
at step 5, the operation of step 4 is repeated until the load is fully dried.

In this method, the autoclave is preheated by using the heating apparatus to reduce condensation of the water steam filled in sterilization stage; during a drying process, the air-blowing apparatus stirs the air current in the autoclave such that the heat on the autoclave can be more easily transferred to the load under the action of the flowing air, so as to speed up water evaporation. Therefore, the drying efficiency and effect can be improved without increasing the vacuuming degree or heating temperature or extending the drying time.

The above descriptions are made only to the specific embodiments of the present disclosure and the scope of protection of the present disclosure is not limited hereto. Any changes or substitutions easily thought of by those skilled in the art within the technical scope of the present disclosure shall fall within the scope of protection of the present disclosure. Therefore, the scope of protection of the present disclosure should be indicated by the scope of protection of the claims.

## Claims

1. A steam sterilizer, wherein it comprising a sterilizer body (1), and a drying system disposed inside the sterilizer body (1); the drying system comprises an autoclave (2), and the autoclave (2) is connected with a heating apparatus, a steam apparatus, a vacuuming apparatus and a return air apparatus; the heating apparatus is used to heat the autoclave (2); the steam apparatus is used to fill steam into the autoclave (2); the vacuuming apparatus is used to vacuum the autoclave (2), and the return air apparatus is used to return air into the vacuumed autoclave (2); the sterilizer body (1) is internally provided with an air-blowing apparatus (3) used to stir an air current in the autoclave (2).

2. The steam sterilizer of claim 1, wherein the air-blowing apparatus (3) comprises a drive unit (4) and fan blades (5); the drive unit (4) is disposed outside the autoclave (2) and the fan blades (5) are rotatably connected inside the autoclave (2); the drive unit (4) is connected with the fan blades (5) in transmission manner to drive the rotation of the fan blades (5).

3. The steam sterilizer of claim 2, wherein a first magnetic coupling (6) is rotatably connected outside the autoclave (2) and a second magnetic coupling (7) is rotatably connected at a position corresponding to the first magnetic coupling (6) inside the autoclave (2); the drive unit (4) is connected to the first magnetic coupling (6) and the second magnetic coupling (7) is connected to the fan blades (5); the drive unit (4) is used to drive the first magnetic coupling (6) to rotate, and the first magnetic coupling (6) is magnetically attracted with the second magnetic coupling (7) to drive the second magnetic coupling (7) and the fan blades (5) to rotate.

4. The steam sterilizer of claim 3, wherein each of the first magnetic coupling (6) and the second magnetic coupling (7) comprises a base (8), a magnet (9) and a rotary shaft (10); the base (8) is fixedly mounted on the autoclave (2); a bearing (12) is disposed on the base (8); the rotary shaft (10) is rotatably connected to the bearing (12); the magnet is connected to the rotary shaft (10) to rotate along with the rotary shaft (10).

5. The steam sterilizer of claim 4, wherein there are six magnets (9) which are annularly distributed on an outer wall of the rotary shaft (10).

6. The steam sterilizer of claim 5, wherein the autoclave (2) is an autoclave (2) made of a non-magnetic material.

7. The steam sterilizer of claim 6, wherein the magnets (9) are magnets (9) resistant to high temperature, and the bearing (12) on the second magnetic coupling (7) is a ceramic bearing (12).

8. The steam sterilizer of claim 7, wherein the fan blades (5) are externally sleeved with a protective hood (11) with hollow design.

9. A sterilization drying method for the steam sterilizer of any one claim of claims 1-8, wherein it comprises the following steps:
step 1, preheating stage: the heating apparatus heats and controls the outer wall of the autoclave to a particular temperature, and meanwhile the air-blowing apparatus stirs the air current in the autoclave until the temperature in the autoclave reaches a preset temperature;
step 2, sterilization stage: the vacuuming apparatus vacuums the autoclave, and then the steam apparatus fills high-temperature water steam into the autoclave and holds for a period of time to achieve sterilization effect;
step 3, water and air drainage stage: the vacuuming apparatus vacuums the autoclave to drain air and water steam in the autoclave;
step 4, drying stage: after the vacuuming apparatus vacuums the autoclave to a set vacuum degree, the vacuuming apparatus is turned off, and the return air apparatus is turned on to allow a large amount of bacteria-filtered air to enter the autoclave; the air in the autoclave is stirred to circulate by the air-blowing apparatus, such that the heating apparatus quickly heats the interior of the autoclave and a load in the autoclave until the temperature in the autoclave reaches a set temperature, and then the vacuuming apparatus is turned on again to perform vacuuming;
step 5, the operation of step 4 is repeated until the load is fully dried.
